# EUROPÄISCHE PATENTANMELDUNG

(11) **EP 2 208 460 A1**
(43) Veröffentlichungstag der Anmeldung: **21.07.2010**
(21) Anmeldenummer: 09150848.1
(22) Anmeldetag: 19.01.2009
(51) Int. Cl.: A61B 5/00

(54) **Erkennung und Lokalisierung von Gewebe durch Lichtanalyse**

(71) Anmelder: BrainLAB AG, 85622 Feldkirchen (DE)
(72) Erfinder: Druse, Alexander, 80637, München (DE); Urban, Alexander, 85661, Forstinning (DE); Vollmer, Fritz, 80469, München (DE)
(74) Vertreter: Schwabe - Sandmair - Marx

(57) **Zusammenfassung**

Die Erfindung betrifft ein Verfahren zur Erkennung und Lokalisierung von Körpergewebe, bei dem ein Lichtstrahl (6), der von einem interessierenden Gewebepunkt oder Gewebebereich (4) ausgeht, einerseits mit Hilfe eines optischen, medizintechnischen Trackingsystems (2) positionell bestimmt wird, und andererseits bezüglich seiner Lichteigenschaften analysiert wird, um hieraus die Natur des Gewebepunktes oder Gewebebereichs zu bestimmen. Ferner betrifft sie eine Vorrichtung zum Erkennen und Lokalisieren von Körpergewebe, mit einem optischen, medizintechnischen Trackingsystems (2), das einen Lichtstrahl (6) erfasst, der von einem interessierenden Gewebepunkt oder Gewebebereich (4) ausgeht, und den Ort des Gewebepunktes oder Gewebebereichs (4) positionell bestimmt, und mit einem Lichtanalysator, der den Lichtstrahl bezüglich seiner Lichteigenschaften analysiert, um hieraus die Natur des Gewebepunktes oder Gewebebereichs zu bestimmen.

## Beschreibung

Die vorliegende Erfindung betrifft die Erkennung und Lokalisierung von Gewebe für medizintechnische Zwecke. Hierzu werden ein Verfahren und eine Vorrichtung vorgestellt.

Die Erkennung und Lokalisierung von Gewebe bzw. Gewebeteilen, wie sie Gegenstand der vorliegenden Erfindung ist, hat unter anderem eine hohe Bedeutung bei der Erfassung von Abgrenzungen bzw. Grenzen und Abmessungen von Tumoren an einem Gewebe in einem interessierenden Gebiet. Beispielsweise wird gemäß dem Stand der Technik wie er aus der US 5,699,798 bekannt ist, ein interessierendes Gebiet mit einer Leuchtquelle ausgeleuchtet, die elektromagnetische Strahlung mindestens einer Wellenlänge abstrahlt, welche mit einem Farbstoff interagiert. Die elektromagnetische Strahlung hat eine Wellenlänge von etwa 450 nm bis ungefähr 2500 nm.

Ein weiterer Ansatz gemäß der EP 1 795 142 B1 ist eine Kombination eines medizintechnischen Trackingsystems mit Video- oder Infrarotkamerasystem und mindestens einer lokalisierbaren Gamma-Kamera zum Detektieren von Gammastrahlung, welche von einem Tracer-Material emittiert wird.

Beide genannten Verfahren gemäß dem Stand der Technik sind aufwendig (Einfärben des Gewebes; Gamma-Kamera).

Die US 2006/0058683 A1 beschreibt ein optisches Untersuchungssystem für biologisches Gewebe ohne Lokalisation.

Es ist die Aufgabe der vorliegenden Erfindung, die Erkennung und Lokalisierung von Gewebe oder Gewebeteilen bzw. Gewebebereichen/-punkten zu optimieren.

Insbesondere soll die Information über das Gewebe positionsgenau und ohne zu großen Aufwand zur Verfügung gestellt werden.

Diese Aufgabe wird erfindungsgemäß durch ein Verfahren gemäß dem Anspruch 1 sowie eine Vorrichtung gemäß dem Anspruch 9 gelöst. Die Unteransprüche definieren bevorzugte Ausführungsformen der Erfindung.

Erfindungsgemäß wird also ein Verfahren zur Erkennung und Lokalisierung von Körpergewebe bereitgestellt, bei dem ein Lichtstrahl, der von einem interessierenden Gewebepunkt oder Gewebebereich ausgeht (z. B. durch Reflexion eines auf das Gewebe auftreffenden Lichtstrahls oder durch angeregte Lichtemission des Gewebes oder eines im Gewebe angereicherten Farbstoffes, wie z. B. Fluoreszenz oder Phosphoreszenz), einerseits mit Hilfe eines optischen, medizintechnischen Trackingsystems positionell bestimmt wird und andererseits bezüglich seiner Lichteigenschaften analysiert wird, um hieraus die Natur des Gewebepunktes oder Gewebebereichs zu bestimmen. Hierzu verwendete Vorrichtungsteile umfassen das Trackingsystem insbesondere einen Lichtempfänger, und einen Lichtanalysator, zur Feststellung der Eigenschaften des vom Gewebe kommenden Lichtes.

Gemäß der Erfindung wird somit das Licht, das von dem Gewebe oder dem Gewebepunkt ausgeht, gleichzeitig auf seine Beschaffenheit geprüft und der Ort, von dem das Licht ausgeht, positionell bestimmt. Mit anderen Worten wird erfindungsgemäß der Lichtreflex oder der angeregte Lichtstrahl, der das Gewebe wieder verlässt, vorrichtungsgestützt analysiert, um Rückschlüsse auf die Eigenschaften des Gewebes treffen zu können. Dazu muss, das Gewebe nicht unbedingt vorher speziell behandelt werden (z.B. Einfärbung), und es sind auch keine aufwendigen Vorrichtungen wie Gamma-Kameras notwendig. Man wird entweder wissen, welche Art von Umgebungslicht auf das Gewebe auftrifft (z.B. Beleuchtung im Operationssaal) oder definiertes Licht auf den Bereich aufstrahlen (z. B. Laserlicht oder IR-Licht einer IR-Trackingkamera des Navigationssystems), um so aus der Reflexion/Emission Schlüsse hinsichtlich der Gewebebeschaffenheit an einzelnen, lokalisierbaren Bereichen ziehen zu können. Damit wird es möglich, Grenzen und Abmessungen interessierender Bereiche, beispielsweise von Tumorgewebe, einfach zu detektieren. Die Abgrenzung von beispielsweise Tumorgewebe kann auch durch Substanzen definierter Emissions-/Reflexionseigenschafte erfolgen die sich durch ihre biologischen Eigenschaften beispielsweise in Tumorgewebe anreichern. Mit einer solchen Detektion kann dann wiederum eine Information über zu behandelnde Gebiete erhalten oder aktualisiert werden, so dass bei der Behandlung gesunde Gewebeteile geschont und kranke Gewebeteile entfernt oder behandelt werden können. Die Genauigkeit der Ortserfassung, die mit einem medizintechnischen Trackingsystem verbunden ist, kommt dabei unmittelbar dem Patienten zu Gute. Präoperative Datensätze können aktualisiert bzw. mit den Gewebeinformationen ergänzt werden. In vielen Operationssälen stehen schon medizintechnische Trackingsysteme zur Verfügung, so dass die Erfindung leicht in vorhandene Hardware eingebunden werden kann.

Der Lichtstrahl, der von dem Gewebepunkt oder dem Gewebebereich ausgeht (der Lichtstrahl kann ein sehr schmaler Lichtstrahl oder ein flächiger bzw. ein Lichtstrahl mit größerem Querschnitt sein), kann anhand einer Eigenschaft wie Wellenlänge, Leuchtstärke, Kohärenz, Richtung, Streuung oder einer Kombination solcher Eigenschaften analysiert werden. Grundsätzlich eignet sich jede Eigenschaft des Lichtes, die sich durch die zu bestimmenden Gewebeeigenschaften oder durch eingebrachter Farbstoffe verändert und die mit einer entsprechenden Analysevorrichtung nachgewiesen werden kann.

Bei einer Ausführungsform der vorliegenden Erfindung wird ein Quellenlichtstrahl mit definierten, bekannten Lichteigenschaften auf den Gewebepunkt oder Gewebebereich aufgestrahlt, und der Unterschied zwischen den Eigenschaften des Quellenlichtstrahls und des Lichtstrahls, der eingangs genannt wurde (der vom Gewebe ausgeht bzw. reflektiert wird) wird der Bestimmung der Natur des Gewebepunktes oder Gewebebereichs zugrundegelegt. Der Vorteil ist dabei, dass man die Eigenschaften des Quellenlichtstrahls genau kennen bzw. einstellen kann, um damit auch sehr hochwertige Informationen über das Gewebe aus dem reflektierten Strahl zu entnehmen. Der Quellenlichtstrahl kann ein Laserlichtstrahl sein, insbesondere im infraroten, ultravioletten oder sichtbaren Lichtwellenlängenbereich. Er kann flächig auf dem Gewebe auftreffendes Licht mit bekannten Eigenschaften umfassen und/oder einen Punktlichtstrahl. Beispielsweise kann auch das IR-Licht der IR-Trackingkamera des Navigationssystems als Quellenlichtstrahl verwendet werden. Bei Verwendung von Punktlichtstrahlen kann die Positionsbestimmung mittels einfacher Triangulation aus dem Trackingsystem bestimmt werden. Bei Verwendung von Flächenlichtstrahlern erhält man flächig leuchtende Strukturen deren Raumposition über spezielle stereoskopische Videoauswertungen erfasst werden kann, wobei zwei stereoskopisch angebrachte Kameras Verwendung finden.

Gemäß einer Variante des erfindungsgemäßen Verfahrens findet die Analyse des Lichtstrahls auf der Basis von lichtverändernden Merkmalen des Gewebepunktes oder Gewebebereichs statt. Die Grenzen und/oder die Ausdehnungen von definierten Gewebeabschnitten können positionell und bezüglich der Gewebeeigenschaften erfasst und die erfassten Daten einem medizintechnischen Navigationssystem zur Verfügung gestellt werden, wobei sie insbesondere zur Aktualisierung der präoperativen Bilddaten genutzt werden.

Die erfindungsgemäße Vorrichtung zum Erkennen und Lokalisieren von Körpergewebe umfasst ein optisches, medizintechnisches Trackingsystem, das einen einzelnen Lichtstrahl oder flächig abgestrahlte Lichstrahlen erfasst, die von einem interessierendem Gewebepunkt oder Gewebebereich ausgehen. Das Trackingsystem bestimmt den Ort des Gewebepunktes oder Gewebebereichs positionell, und die Vorrichtung weist ferner einen Lichtanalysator auf, der den Lichtstrahl bezüglich seiner Lichteigenschaften analysiert, um hieraus die Natur des Gewebepunktes oder Gewebebereichs zu bestimmen.

Die Vorrichtung kann einen Quellenlichtstrahl umfassen, insbesondere einen vorgenannten Laserlichtstrahler, und zwar als Punktlichtstrahler und/oder als Flächenlichtstrahler.

Bei einer erfindungsgemäßen Ausführungsform der Vorrichtung ist der den Lichtstrahl empfangende Teil, insbesondere ein Lichtsensor, speziell ein Faserlichtsensor, am Quellenlichtstrahler angeordnet, welcher wiederum positionell durch das Trackingsystem bestimmt und/oder verfolgt wird.

Die Daten über die Eigenschaften des Lichtstrahl und des Quellenlichtstrahls, d.h. die Gewebenatur und die Position des Gewebepunktes, können in einer Datenverarbeitungseinheit verarbeitet werden, die insbesondere ein Teil eines medizintechnischen Navigationssystems ist.

Die Erfindung wird im Weiteren anhand von Ausführungsbeispielen und unter Bezugnahme auf die beiliegenden Zeichnungen näher erläutert. Sie kann alle hierin beschriebenen Merkmale einzeln sowie in jedweder sinnvollen Kombination umfassen und insbesondere auch als Programm ausgestaltet sein, das, wenn es auf einem Computer läuft oder in einem Computer geladen ist, den Computer veranlasst, ein Verfahren durchzuführen, wie es oben in verschiedenen Ausführungsformen beschrieben worden ist. Die Erfindung umfasst ferner ein Computerprogramm-Speichermedium, das ein solches Programm aufweist.

### In den beiliegenden Zeichnungen zeigen:

- Figur 1: eine schematische Darstellung eines Vorrichtungs-Setups, welches bei der Umsetzung der vorliegenden Erfindung verwendet wird; und
- Figur 2: einen interessierenden Gewebebereich mit erfindungsgemäßer Lichtbestrahlung/Reflexion.

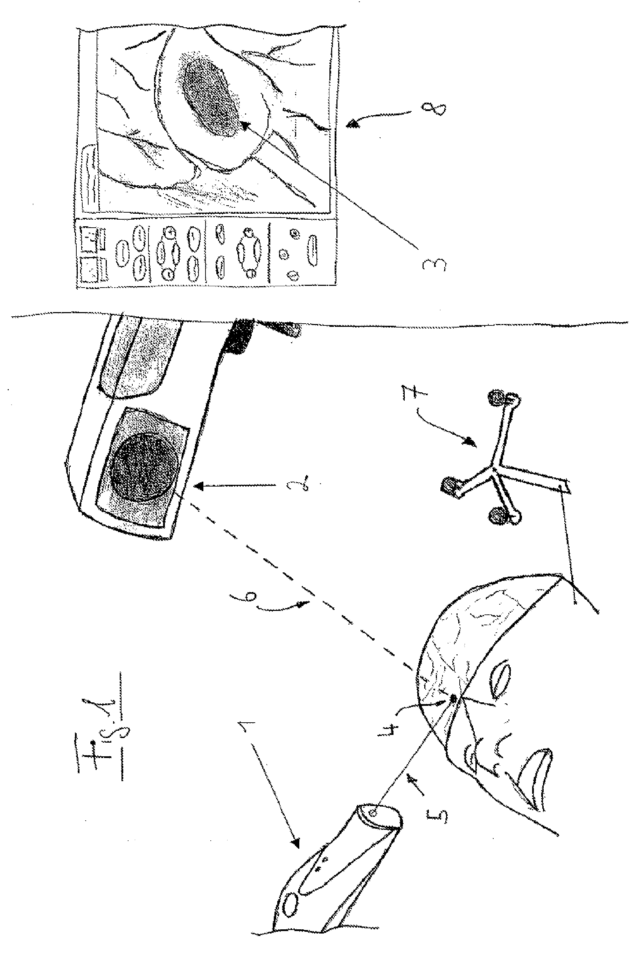

Die in der Figur 1 dargestellten Vorrichtungsteile sind ein Laser-Punktstrahler 1, ein medizintechnisches Trackingsystem 2 mit Kamera und eine Referenzanordnung 7, die mit dem Kopf eines Patienten verbunden ist und so dessen Positionserfassung mithilfe des Trackingsystems 2 gestattet. Ebenso kann das Trackingsystem 2 einen Lichtpunkt (Reflexionspunkt 4) erfassen, dessen Lichtstrahl zur Kameralinse mit dem Bezugszeichen 6 worden ist. Weil das Trackingsystem 2 zwei Kameras aufweist und damit zwei Lichtstrahlen 6 erfassen kann, kann die Position des Lichtpunktes 4 dreidimensional erfasst werden, und aufgrund der Positionserfassung des Patienten über die Referenzanordnung 7 ist dann auch bekannt, wo der Punkt 4 am Patienten liegt.

Der Lichtpunkt 4 entsteht durch das Aufstrahlen eines Lichtstrahls 5 mittels des integrierten Laserstrahlers 1, der im vorliegenden Fall ein Handlaserstrahler ist.

Auf der rechten Seite der Figur 1 ist abgegrenzt noch die Bildschirmausgabe 8 eines Navigationssystems dargestellt, wobei das Bezugszeichen 8 auch das Navigationssystem samt Bildschirmausgabe bezeichnen kann. Das Bild 3 ist ein Bild, welches mit Hilfe der Informationen aus dem erfindungsgemäßen Verfahren erzeugt wurde und dem Chirurgen unterstützend zur Verfügung steht.

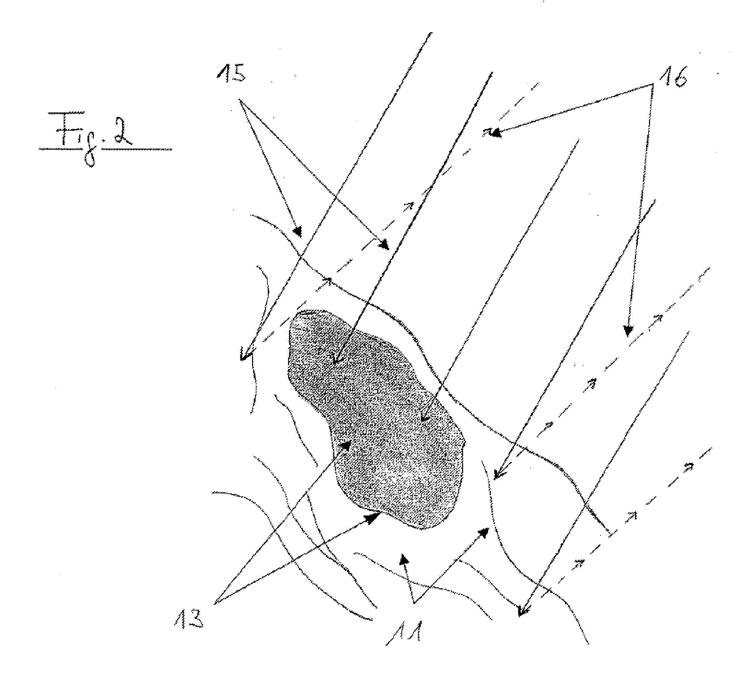

Die Bezugszeichen der Figur 2 bezeichnen bei 11 einen interessierenden Gewebebereich; 13 weist auf einen Tumorabschnitt und dessen Begrenzung hin. Mit 15 sind einfallende Lichtstrahlen (Quellenlicht) bezeichnet, während das Bezugszeichen 16 auf reflektiertes Licht hinweist, das erfindungsgemäß "ausgelesen" werden kann.

Der in Figur 1 dargestellte integrierte Laser 1 kann einen oder mehrere Laserstrahlen 5 von definierter Wellenlänge aussenden. Der reflektierte Laserstrahl 6 von dem Punkt 4 wird analysiert, und hierzu zunächst durch die Kameras des Trackingsystems 2 aufgefangen, Mit einer Software des Navigationssystems wird der Strahl 6 analysiert, d.h. die Software erkennt die Eigenschaften und Zusammensetzung des reflektierten/emittierten Lichts, das vom Gewebe abgestrahlt wird, wodurch erkannt werden kann, welche Art von Gewebe am Punkt 4 vorliegt. Dadurch kann (während der Operation) vorher erfasstes Datenmaterial aktualisiert werden, und zwar auch mehrmals hintereinander, so oft dies notwendig ist.

Es ist möglich, Laserlicht aufzustrahlen, das mehrere Wellenlängen abdeckt (z.B. Laserlicht 15 in Figur 2), um zu überprüfen, wie unterschiedliche Wellenlängen reflektiert werden (Reflexion 16 in Figur 2). Aus diesen Informationen können dann die Positionen des Tumorgewebes 13 und seine Abgrenzung zum umgebenden gesunden Gewebe 11 erkannt werden. Natürlich funktioniert dies einerseits mit einem flächig aufgebrachten Quellenlichtstrahl oder einem Quellenlichtstrahl mit größerem Durchmesser, andererseits aber auch mit einem Punktlichtstrahl (Figur 1) der über das Gewebe bewegt wird und dessen Reflektionen nacheinander analysiert werden.

Wie schon vorher bemerkt kann der Laser im Infrarotbereich, im Ultraviolettbereich oder im sichtbaren Lichtbereich arbeiten. Eine interessante Ausgestaltung einer Vorrichtung gemäß der Erfindung umfasst eine starre Faseroptik, die an einem Handgriff angeordnet ist, wie er beispielsweise in Figur 1 mit dem Bezugszeichen 1 bezeichnet worden ist. Dieser Handgriff wäre aber in diesem Fall mit einer Trackingreferenz für das Trackingsystem 2 versehen und gestattet so die Navigation bzw. das Tracking der Faserenden. Diese Fasern bzw. Faserenden werden verwendet, um das Quellenlicht zum Beleuchten des Gewebes, das untersucht wird, zu leiten, während sie selbst oder auch andere Fasern im Vorderteil des Handgriffs andererseits auch das zurückgestrahlte Licht empfangen und es an die Detektorvorrichtung/Analysevorrichtung weitersenden. Somit können auch schwache Signale/Lichtveränderungen des zu untersuchenden Gewebes ausgewertet werden.

Mit dem erfindungsgemäßen Verfahren bzw. der erfindungsgemäßen Vorrichtung ist es möglich, nicht nur Tumoren und ihre Grenzen zu erfassen, sondern auch andere Oberflächenstrukturen wie z.B. Knochenoberfläche oder Muttermale.

## Patentansprüche

1. Verfahren zur Erkennung und Lokalisierung von Körpergewebe, bei dem ein Lichtstrahl (6), der von einem interessierenden Gewebepunkt oder Gewebebereich (4) ausgeht, einerseits mit Hilfe eines optischen, medizintechnischen Trackingsystems (2) positionell bestimmt wird, und andererseits bezüglich seiner Lichteigenschaften analysiert wird, um hieraus die Natur des Gewebepunktes oder Gewebebereichs zu bestimmen.

2. Verfahren nach Anspruch 1, bei dem der Lichtstrahl anhand einer Eigenschaft wie
- Wellenlänge,
- Leuchtstärke,
- Kohärenz,
- Richtung,
- Streuung,
oder einer Kombination solcher Eigenschaften analysiert wird.

3. Verfahren nach Anspruch 1 oder 2, bei dem ein Quellenlichtstrahl (5) mit definierten, bekannten Lichteigenschaften auf den Gewebepunkt oder Gewebebereich (4) aufgestrahlt wird und der Unterschied zwischen den Eigenschaften des Quellenlichtstrahls (5) und des Lichtstrahls (6) der Bestimmung der Natur des Gewebepunktes oder Gewebebereichs zugrunde gelegt wird.

4. Verfahren nach Anspruch 3, bei dem der Quellenlichtstrahl (5) ein Laserlichtstrahl ist, insbesondere im infraroten, ultravioletten oder sichtbaren Lichtwellenlängenbereich.

5. Verfahren nach Anspruch 3 oder 4, bei dem der Quellenlichtstrahl ein flächig auf dem Gewebe auftreffendes Licht mit bekannten Eigenschaften ist.

6. Verfahren nach Anspruch 3 oder 4, bei dem der Quellenlichtstrahl ein Punktlichtstrahl (5) ist.

7. Verfahren nach einem der Ansprüch 1 bis 6, bei dem die Analyse des Lichtstrahls auf der Basis von lichtverändernden, insbesondere körpereigenen Merkmalen des Gewebepunktes oder Gewebebereichs (4) stattfindet.

8. Verfahren nach einem der Ansprüche 1 bis 7, bei dem Grenzen und/oder Ausdehnungen von definierten Gewebeabschnitten positionell und bezüglich der Gewebeeigenschaften erfasst und die erfassten Daten einem medizintechnischen Navigationssystem zur Verfügung gestellt werden, wobei sie insbesondere zur Aktualisierung vorher erfassten Datenmaterials genutzt werden.

9. Vorrichtung zum Erkennen und Lokalisieren von Körpergewebe, mit einem optischen, medizintechnischen Trackingsystems (2), das einen Lichtstrahl (6) erfasst, der von einem interessierenden Gewebepunkt oder Gewebebereich (4) ausgeht, und den Ort des Gewebepunktes oder Gewebebereichs (4) positionell bestimmt, und mit einem Lichtanalysator, der den Lichtstrahl bezüglich seiner Lichteigenschaften analysiert wird, um hieraus die Natur des Gewebepunktes oder Gewebebereichs zu bestimmen.

10. Vorrichtung nach Anspruch 9, **dadurch gekennzeichnet, dass** sie einen Quellenlichtstrahler (1) umfasst, insbesondere einen Laserlichtstrahler, der speziell im infraroten, ultravioletten oder sichtbaren Lichtwellenlängenbereich strahlt.

11. Vorrichtung nach Anspruch 10, **dadurch gekennzeichnet, dass** der Quellenlichtstrahler ein Punktlichtstrahler (1) ist.

12. Vorrichtung nach Anspruch 10, **dadurch gekennzeichnet, dass** der Quellenlichtstrahler ein Flächenlichtstrahler ist.

13. Vorrichtung nach einem der Ansprüche 10 bis 12, **dadurch gekennzeichnet, dass** der den Lichtstrahl (6) empfangende Teil, insbesondere ein Lichtsensor, speziell ein Faserlichtsensor, am Quellenlichtstrahler (1) angeordnet ist, welcher wiederum positionell durch das Trackingsystem (2) bestimmt und/oder verfolgt wird.

14. Vorrichtung nach einem der Ansprüche 10 bis 13, **dadurch gekennzeichnet, dass** die Daten über die Eigenschaften des Lichtstrahls (6) und des Quellenlichtstrahls (5) in einer Datenverarbeitungseinheit verarbeitet werden, die insbesondere ein Teil eines medizintechnischen Navigationssystems ist.

15. Programm, das, wenn es auf einem Computer läuft oder in einem Computer geladen ist, den Computer veranlasst, ein Verfahren gemäß einem der Ansprüche 1 bis 8 durchzuführen.

16. Computerprogramm-Speichermedium, das ein Programm nach Anspruch 15 aufweist.
